# EUROPEAN PATENT APPLICATION

(11) **EP 4 397 270 A1**
(43) Date of publication of application: **10.07.2024**
(21) Application number: 23150313.7
(22) Date of filing: 04.01.2023
(51) Int. Cl.: A61B 34/20, A61B 90/00

(54) **TECHNIQUE FOR DETERMINING AN OBJECT MARKER ARRANGEMENT**

(71) Applicant: Stryker European Operations Limited, Carrigtwohill, Co. Cork T45 HX08 (IE)
(72) Inventor: HORNECKER, Patrick, 79356 Eichstetten (DE)
(74) Representative: Röthinger, Rainer

(57) **Abstract**

A technique for determining an object marker arrangement comprising a plurality of object markers arranged on at least two non-parallel surfaces or non-parallel surface portions of an object is provided. The object marker arrangement is characterized by positions of the object markers, wherein a reference device with a pre-determined reference pattern is provided. A method implementation of the technique comprises several steps at least partially performed by a processing device. In more detail, the method comprises the step of receiving image data representative of a plurality of images that contain the reference pattern and at least a subset of the object markers, wherein at least some of the images were captured by an imaging device from different viewing angles. The reference pattern and the object markers were arranged in a fixed spatial relationship relative to each other when the images were captured. The method further comprises determining positions of the object markers relative to the reference pattern, wherein the position of an individual one of the object markers is determined based on at least two images that contain the individual object marker and based on geometrical information about the reference pattern.

## Description

### Technical Field

The present disclosure generally relates to a technique for determining an arrangement of a plurality of markers on an object. The object marker arrangement may be used for tracking a surgical object. The technique may be implemented in the form of a method, a computer program product, a processing device and a system.

### Background

In surgical navigation scenarios, it is common to attach trackers to patients, surgical instruments or other objects. The trackers typically have a known spatial relationship to the object. Once a spatial position of a tracker has been determined by a tracking system, the spatial position of the object is typically known also and can be tracked.

A conventional optical tracker carries a spatially predetermined arrangement of markers that can individually be detected by a tracking camera of the tracking system. Knowledge of the pre-determined marker arrangement is required to identify the markers in image data taken by the tracking camera and to track their movement in space.

The marker arrangement is often defined at a manufacturing site for a particular tracker type and, therefore, is known to the tracking system. In some scenarios however, for example when there are large manufacturing tolerances, the relative positions of the markers are not known in advance. In such cases, the marker arrangement has to be determined for each tracker individually.

One way to determine the marker arrangement is to hold a trackable pointer to each marker of a tracker and instruct the tracking system to determine each marker position based on the position of the tracked pointer. The approach of using a tracked pointer may be cumbersome for the user and time consuming. Furthermore, the accuracy of determining the marker arrangement depends on the user's ability to align the pointer with the markers of the tracker.

### Summary

There is a need for an efficient technique for determining an object marker arrangement in the above and other scenarios.

According to a first aspect, a method for determining an object marker arrangement comprising a plurality of object markers arranged on at least two non-parallel surfaces or non-parallel surface portions of an object is provided. The object marker arrangement is characterized by positions of the object markers, wherein a reference device with a pre-determined reference pattern is provided. The method comprises several steps at least partially performed by a processing device. The method comprises receiving image data representative of a plurality of images that contain the reference pattern and at least a subset of the object markers, wherein at least some of the images were captured by an imaging device from different viewing angles. The reference pattern and the object markers were arranged in a fixed spatial relationship relative to each other when the images were captured. The method further comprises determining positions of the object markers relative to the reference pattern, wherein the position of an individual one of the object markers is determined based on at least two images that contain the individual object marker and based on geometrical information about the reference pattern.

The arrangement of object markers is characterized by positions of the object markers. These positions may be defined relative to each other (e.g., in the form of vectors or by their Euclidean distances) or as coordinates in an object marker coordinate system or any other coordinate system. The object markers may be configured to be detected optically when taking the images. As an example, the object markers may have light-reflecting or light-emitting properties (e.g., in the visible or infrared light spectrum). The object marker arrangement may comprise more than 3, 4, 5, 6, 8, 10 or 15 object markers.

The reference pattern may include reference markers. The reference markers may be configured to be detected optically when taking the images. The reference markers may have similar optical properties like the object markers. The geometrical information about the reference pattern may include positions of the reference markers (e.g., in the form of relative positions, Euclidean distances or as coordinates in a reference coordinate system).

The object markers are arranged on at least two non-parallel surfaces or non-parallel surface portions of the object. The non-parallel surfaces or non-parallel surface portions may be located at an angle relative to each other (e.g., of more than 20°, more than 45° or more than 60°). The object markers may be arranged on three, four, five, six or more non-parallel surfaces. At least one or more of the object markers may be arranged on a curved surface. For example, the curved surface may define the non-parallel surface portions.

The images were captured by the imaging device from different viewing angles. The different viewing angles may comprise at least a first viewing angle in which the individual object marker is visible and a second viewing angle in which the individual object marker is not visible. In the second viewing angle at least another one of the object markers may be visible. In each of the first viewing angle and the second viewing angle at least three object markers may be visible, so that in total six or more object markers may be in use. Each viewing angle may be associated with a dedicated one of the non-parallel surfaces or non-parallel surface portions on which the markers are arranged.

The image data may have been taken while the imaging device was moving relative to the object marker arrangement. The imaging device may comprise one or more cameras or camera modules. The imaging device may comprise a stereo camera capable of taking two images at substantially the same time and under substantially the same viewing angle. The imaging device may be configured to capture light in the visible or infrared spectrum. The imaging device may be a video camera (e.g., a webcam) capturing video data comprising the image data.

Determining the position of the individual object marker may comprise determining, based on at least a first image and a second image of the plurality of images that contain the reference pattern and the individual object marker, a position of the individual object marker relative to the reference pattern (e.g., in a coordinate system of the reference device). In case the imaging device takes the form of a stereo camera comprising a first camera module and a second camera module, the first and second images may be taken by the first camera module and second camera module, respectively. The resulting two images (plus, optionally, supplemental information about one or more of the arrangement of the two camera modules relative to each other, a viewing axis of each camera module, the dimensions of the object markers, etc.) and the geometrical information about the reference pattern may then be used to determine the position of the individual object marker relative to the reference device.

Another one of the individual object markers may not be contained in at least one of the first image and the second image (for example because it is arranged on another surface or surface portion than one or more of further object markers and is thus not visible in the viewing angle at which at least one of the first image and the second image were taken). In such a case the method may comprise determining, based on at least a third image and one of the first, the second and a fourth image of the plurality of images that contain the reference pattern and the other one of the object markers, a position of the other one of the object markers relative to the reference pattern (e.g., in the coordinate system of the reference device).

The object marker arrangement may be determined in an object marker coordinate system. In some variants, the positions of the object markers as initially determined in the reference coordinate system may be transferred into object marker coordinate system. An origin of the object marker coordinate system may have a predefined geometric relationship to one of the object markers. For example, the object marker coordinate system may have its origin at the position of one of the object markers or at a predefined offset relative to that position.

At least one of the object markers may have a rotational-symmetric shape (e.g., the shape of a circle or a regular polygon). One or more of the object markers may be configured to be handled (e.g., attached to or removed from the object) separately from one or more other object markers. One or more of the object markers may be substantially planar. One or more of the object markers may be arranged on a flexible substrate, such as a foil or a sheet of paper. At least one of the object markers may comprise a reflective material configured to reflect light of at least one of the visible and infrared spectrum.

The method may further comprise removing the reference device from the fixed spatial relationship with the object markers. The removal may in particular take place once the object marker arrangement has been determined. The reference device may be moved out of a field of view of the imaging device. The object may be tracked based on the object marker arrangement after the reference device has been removed.

The method may comprise receiving tracking image data representative of the object markers. The method may comprise tracking the object based on the tracking image data and information about the object marker arrangement. The information about the object marker arrangement may be sufficient to geometrically associate the object marker coordinate system with the object (e.g., a coordinate system of the object) for tracking purposes. The object marker arrangement may thus serve as an object tracker. The object may not be tracked based on the reference pattern. The tracking image data may be captured by the imaging device or a separate tracking device (e.g., a tracking camera).

The method may comprise selecting the position of one of the object markers as a reference position for an object marker coordinate system and transforming the positions of the other object markers in the object marker coordinate system. As explained above, the object marker coordinate system may have its origin in a predefined spatial relationship with the reference position.

The method may further comprise registering an object geometry of the object with the object marker arrangement or the object marker coordinate system. The object geometry may comprise at least one of a rotation axis of the object, an object tip, and at least a portion of a virtual model of the object. In case the object is a medical imaging device, the object geometry may be one of an imaging plane and an imaging volume.

The method may comprise manually arranging the object markers on the object at not-predefined positions before capturing the plurality of images. The object markers may be arranged individually at the object, one at a time, or in groups of 2, 3 or more. Each object marker may be arranged via an adhesive or a magnetic force at the object.

The method may comprise receiving, from the user, user information indicative of a number of the object markers arranged by him or her on the object. The user information may be used to determine the object marker arrangement (e.g., to verify that the complete object marker arrangement has been determined).

According to a second aspect, a computer program product is provided. The computer program product comprises instructions that, when executed on at least one processor, cause the at least one processor to carry out any the method aspects described herein.

The computer program product may be stored on a non-transitory data storage. The non-transitory data storage may comprise at least one of a hard drive, a compact disc, a memory card, and a cloud computing resource.

According to a third aspect, a processing device for determining an object marker arrangement comprising a plurality of object markers arranged on at least two non-parallel surfaces or non-parallel surface portions of an object is provided. The object marker arrangement is characterized by positions of the object markers, and a reference device with a pre-determined reference pattern is provided. The device is configured to receive image data representative of a plurality of images that contain the reference pattern and at least a subset of the object markers, wherein at least some of the images were captured by an imaging from different viewing angles. The reference pattern and the object markers were arranged in a fixed spatial relationship relative to each other when the images were captured. The processing device is further configured to determine positions of the object markers relative to the reference pattern, wherein a position of an individual one of the object markers is determined based on at least two images that contain the individual object marker and based on geometrical information about the reference pattern.

The device may be further configured to perform any of the method aspects described herein.

Also presented is system comprising the processing device and an imaging device configured to capture the plurality of images while moving relative to the object markers.

### Brief Description of the Drawings

Further details, advantages and aspects of the present disclosure will become apparent from the following embodiments taken in conjunction with the drawings, wherein:
- Fig. 1: shows a system comprising an imaging device, a processing device communicatively coupled to the imaging device, an object marker arrangement and a reference pattern;
- Fig. 2: shows a flow diagram of a method for determining an object marker arrangement;
- Fig. 3A: shows an imaging device capturing first image data from a first viewing angle;
- Fig. 3B: shows an image captured from the first viewing angle;
- Fig. 3C: illustrates an individual object marker in a coordinate system of the reference device, as determined from the image data captured under the first viewing angle;
- Fig. 4A: shows the imaging device capturing second image data from a second viewing angle;
- Fig. 4B: shows an image captured from the second viewing angle;
- Fig. 4C: illustrates another object marker in the coordinate system of the reference device, as determined from the image data captured under the second viewing angle;
- Fig. 5A: illustrates all the object markers in a coordinate system of the reference device;
- Fig. 5B: illustrates a transformation between a coordinate system of the object marker arrangement and the coordinate system of the reference device;
- Fig. 5C: illustrates the object marker arrangement in the object marker coordinate system; and
- Fig. 6: shows exemplary coordinate system transformations in the context of tracking a medical imaging device.

### Detailed Description

In the following description, exemplary embodiments of a method, a computer program product, a processing device and a system for determining an object marker arrangement will be explained with reference to the drawings. The same reference numerals will be used to denote the same or similar structural features.

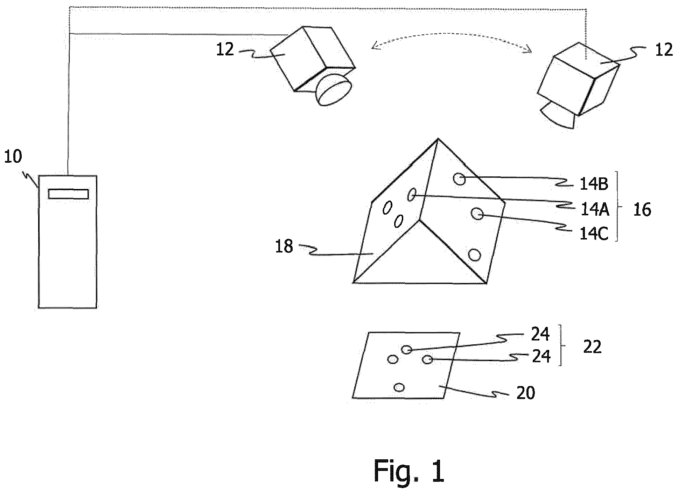

Fig. 1 shows an exemplary implementation of an environment in which embodiments of the present disclosure can be implemented. The environment includes a processing device 10 and an imaging device 12 communicatively coupled to the processing device 10. In some variants, the environment is a surgical environment (e.g., an operating room).

The processing device 10 may be a computer, a server, a tablet or provided at least partially by cloud computing resources. The processing device 10 may be part of, or configured to be communicatively coupled to, a tracking system (that may also comprise the imaging device 12). The tracking system can be a surgical tracking system. The processing device 10 may comprise a non-transitory storage medium storing a computer program product. The computer program product may comprise instructions that, when executed on at least one processor, cause the at least one processor to carry out any the method aspects described herein.

The imaging device 12 may be a configured as a video camera having a sensibility in the infrared or visible light spectrum. In some implementations, the imaging device 12 is configured as a stereo camera with two dedicated camera modules for taking two images at a time, but it may also be realized as a mono camera, such as a regular webcam. The imaging device 12 is configured to be freely movable by a user operating the imaging device 12, or to be movable along a predetermined trajectory.

Fig. 1 also illustrates a plurality of markers 14 arranged on an object 18. The object markers 14 are arranged in a dedicated object marker arrangement 16 on the object 18. The object 18 may be a medical imaging apparatus (such as a computed tomography, CT, scanner, a magnetic resonance imaging, MRI, scanner or a C-arm), a surgical robot, or a surgical table. The object 18 may also be a surgical instrument, such as a clamp, a drill, a saw, a needle, or a suction tube. The object 18 may alternatively be any body part of a patient.

In the scenario illustrated in Fig. 1, the object 18 has several surfaces. The object markers 14 are arranged on two non-parallel surfaces of the object 18. Alternatively, the object markers 14 may be arranged on more than two non-parallel surfaces such as three, four, five, six, or more surfaces. In still further realizations, one or more of the object markers 14 may be arranged on a curved (e.g., convex or concave) surface.

In the configuration of Fig. 1 and in similar configurations, not all object markers 14 may be visible for a given viewing angle of the imaging device 12. In particular, there exist positions and orientations of the imaging device 12 in which certain object markers 14 are visible to the imaging device 12, while others are not. As becomes apparent from Fig. 1, there exists a first viewing angle in which individual object markers, such as an object marker 14A, are visible and a second viewing angle in which the individual object markers, such as the object marker 14A, are not visible. In a second viewing angle different from the first viewing angle, at least another one of the object markers, such as an object marker 14B, is visible. In each of the first viewing angle and the second viewing angle, at least three object markers 14 are visible, but this is not a must.

The object markers 14 have a shape that has at least one of a mirror symmetry and a rotational symmetry. The object markers 14 may have the shape of a circle (as illustrated in Fig. 1), an oval, or a regular polygon (e.g., a regular triangle, square, pentagon, etc.). One or more of the object markers 14 may be active devices configured to emit light (e.g., light emitting diodes, LEDs) or passive devices configured to reflect light. When implemented as passive devices, the object markers 14 may comprise a reflective material configured to reflect light of at least one of the visible and infrared spectrum.

In the implementation of Fig. 1, the object markers 14 have been manually and individually arranged on the object 18 at not-predefined positions, one at a time. To this end, each object marker 14 comprise an attachment structure, such as an adhesive, a magnet, a suction cup, or a portion of a hook-and-loop fastener. The object markers 14 may have been arranged on the object 18 in a detachable manner. In typical use cases, between 6 and 20 object markers 14 may be arranged on the object 18.

Fig. 1 further illustrates a reference device 20 with a pre-determined reference pattern 22. The reference device 20 may be provided only for the purpose of determining the object marker arrangement 16. Alternatively, the reference device 20 may have additional uses such as for tracking an object different from the object 18 on which the object markers 14 are arranged. In such a case, the reference device 20 may take the form of a conventional tracker.

The reference pattern 22 shown in Fig. 1 comprises a plurality of reference markers 24. The reference markers 24 may have a similar configuration as the object markers 14. Alternatively, the reference markers 24 may have a different configuration. If, for example, the object markers 14 are optically passive devices, the reference markers 24 may also be optically active devices (or vice versa). In other examples, the shapes or sizes of the object markers 14 and the reference markers 24 may differ. The object markers 14 and the reference markers 24 may be visible in the same electromagnetic spectrum (e.g., in the infrared spectrum). The reference pattern 22 may in other configurations comprise a single reference marker 24, such as a QR code.

The reference pattern 22 and the object markers 14 are arranged in a fixed spatial relationship relative to each other when images are captured by the imaging device 12. The reference device 20 may be arranged (e.g., laid or attached) on the object 18. Alternatively, the reference device 20 may be arranged on a different object than the object 18 but in the vicinity of the object 18. The reference device 20 may be attached to a patient. The reference device 20 may have an attachment interface (e.g., at least one of an adhesive, a magnet, a clamp, and a hook-and-loop fastener) configured to attach the reference device 20 to the object 18 (or any other object).

The imaging device 12 is configured to capture image data representative of a plurality of images. In certain configurations, at least a subset of the plurality of images each contains the reference pattern 22 and at least one of the object markers 14. Based on images taken from the reference pattern 22 and geometrical information about the reference pattern 22, a position and orientation (i.e., a pose) of the reference pattern 22 can be determined by the processing device 10. This pose may be determined in a base coordinate system (e.g., a coordinate system of the imaging device 12), in which positions of the object markers 14 may be identified also. The geometric information about the reference pattern 22 may define positions of the reference markers 24 (e.g., as Euclidean distances between the reference markers 24 or in a reference marker coordinate system that may have its origin in one of the reference markers 24).

Since the object markers 14 are arranged on at least two inclined surfaces of the object 18 as illustrated in Fig. 1, not every image taken by the imaging device 12 contains every object marker 14. For this reason, the images have to be captured from different viewing angles in order to image each of the object markers 14. The reference device 22 may be used in this context to associate the positions of object markers 14 as derived from the different viewing angles in a single coordinate system.

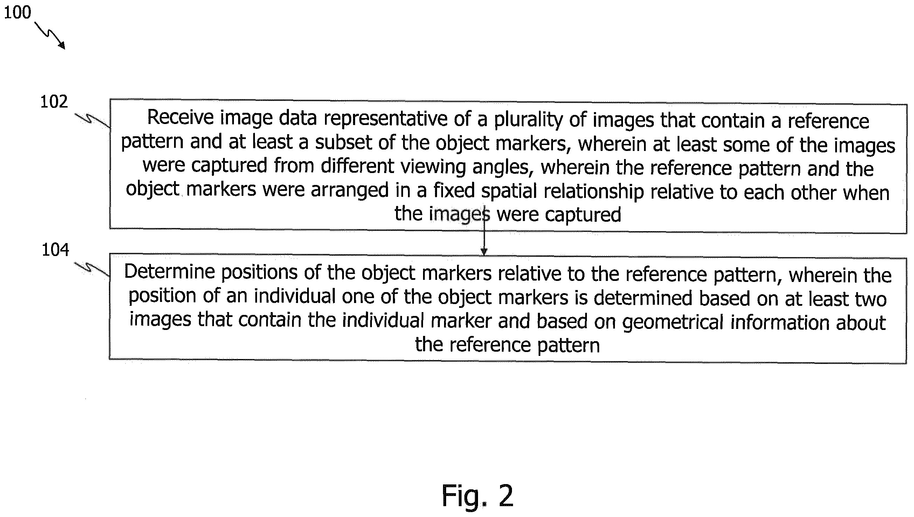

The image data and the geometrical information about the reference pattern 22 allow determining the object marker arrangement 16 using a method that will now be described in more detail with reference to Fig. 2. The resulting object marker arrangement 16, once determined, may then be used further for tracking the object 18.

Fig. 2 shows a flow diagram 100 of a method for determining the object marker arrangement 16. The method may at least in part be performed by the processing device 10 illustrated in Fig. 1.

The method comprises, in step 102, receiving image data representative of a plurality of images. The images each contain the reference pattern 22 and at least a subset (e.g., one, two, three or more) of the object markers 14. The images were captured from different viewing angles (e.g., by a moving imaging device 12 as illustrated in Fig. 1). The reference pattern 22 and the object markers 14 are arranged in a fixed spatial relationship relative to each other when the images are captured.

The method further comprises, in step 104, determining positions of the object markers 14 relative to the reference pattern 22. In more detail, the position of an individual one 14A of the object markers 14 is determined based on at least two images that contain the individual marker 14 and based on geometrical information about the reference pattern 22. The position of the individual one 14A of the object markers 14 can be determined in a coordinate system of the reference device 20.

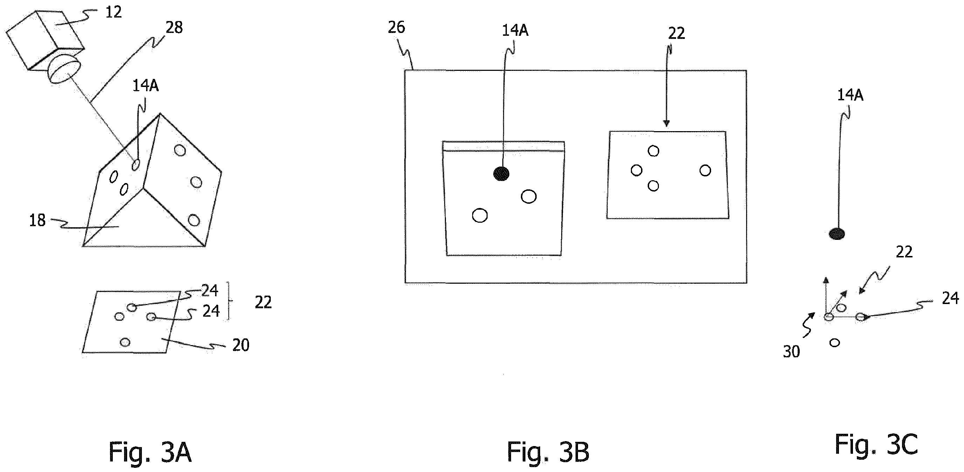

At least two images that contain the individual marker 14A, plus knowledge of the geometrical information about the reference pattern 22, provide a sufficient basis for determining the position of the individual object marker 14A in step 104 relative to the reference device 22. The process of determining the position of the individual object marker 14A involves mathematical and geometrical algorithms and, therefore, can be performed using different approaches that reach the same result. Which approach to use may depend on programming language, data structures and processing power of the processing device 10. In the following, an intuitively accessible way will be described with reference to Figs. 3A to 5C and under the non-limiting assumption that the imaging device 12 is a stereo camera.

Fig. 3A shows the imaging device 12 capturing first image data from a first viewing angle. The resulting first image data is received by the processing device 10 in step 102.

An exemplary content of a first image 26 represented in the first image data is illustrated in Fig. 3B. Since in the present example the imaging device 12 is configured as a stereo camera, the first image data will comprise a second image very similar to the first image 26. The first image 26 and the second image are captured by two separate camera modules, respectively, of the imaging device 12. The two camera modules have a predefined relationship to each other and are both arranged at the first viewing angle relative to the object 18. While the spatial offset between the two camera modules entails that they do not have exactly the same viewing angle, the corresponding difference is negligible in view of fact that both modules capture the same scene (in particular in terms of the object markers 14 visible from the first viewing angle). In one variant, the first viewing angle can be defined as the averaged viewing angle of the two camera modules.

As shown in Fig. 3B, the first image 26 captured from the first viewing angle contains the object markers 14 arranged on a first surface of the object 18, the an individual object marker 14A (emphasized in the drawings with a black marking). The first image 26 also includes the reference pattern 22. Further object markers 14 of the marker arrangement 16 are arranged on a second surface of the object 18, that extends at an exemplary angle of approximately 90° to the first surface. The object markers 14 arranged on the second surface are not visible to the imaging device 12 from the first viewing angle and, therefore, not contained in the first image 26.

Depending on the number of reference markers 24 comprised by the reference device 20, not all reference markers 24 may need to be contained in the first image 26 and the second image. It will suffice in many situations if the same three reference markers 24 are visible in the first image 26 and the second image. The reference device 20, for the purposes of the method illustrated in Fig. 2, may thus be defined by the three or more reference markers 24 contained in the first image 26 and the second image (even if the "real" reference device 20 comprises additional reference markers not contained in the first image 26 and the second image). The number of reference markers 24 visible in the images shall generally be sufficient to define a reference coordinate system in which the object marker positions can be determined in step 104, as will now be described in more detail.

In an initial step, the object markers 14 and the reference markers 24 defining the reference pattern 22 are identified in the first image 26 and in the second image using conventional image processing techniques, such as thresholding by at least one of color, intensity, and intensity gradient. In this initial step, the object markers 14 may not yet be distinguishable from the reference markers 24. Once the object markers 14 and reference markers 24 have been identified in the first image 26 and the second image, their three-dimensional positions in the base coordinate system of the imaging device 12 are determined in a next step. The three-dimensional positions can be determined from the marker positions identified in the two-dimensional image data of the first image 26 and the second image as well as possibly further information. Such further information may comprise one or more of the predefined relationship between the two camera modules that took the first image 26 and the second image, respectively, a direction of an optical axis of each camera module, knowledge about the marker sizes (e.g., diameters), etc..

Once the three-dimensional marker positions in the base coordinate system have been obtained, the object marker positions relative to the reference pattern 22 are determined (step 104 of Fig. 2). In one example, the object marker positions relative to the reference pattern 22 are determined by determining the object marker positions in a reference coordinate system 30 spanned by the reference pattern 22, as illustrated in Fig. 3C. By matching geometrical information about the reference pattern 22 with the marker positions in the base coordinate system, the reference coordinate system 30 is determined in a first step. Then, in a second step, the three-dimensional positions of the object markers 14 visible in the first image 26 and the second image are determined in the reference coordinate system 30 (as exemplarily illustrated for object marker 14A in Fig. 3C).

The geometrical information about the reference pattern 22 may define a first point cloud in space indicative of pre-defined relative positions between the reference markers 24 (e.g., via the Euclidean distances between the reference markers 24). A second point cloud is defined by all marker positions, or at least the positions of the reference markers 24, as determined in the base coordinate system. By matching the first point cloud representative of the reference pattern 22 with the second point cloud representative the marker positions in the base coordinate system, the position and orientation of the reference pattern 22 in the base coordinate system can be determined.

The geometrical information about the reference pattern 22 may further be indicative of how the reference coordinate system is located relative to the reference pattern 22 (e.g., by defining that an origin of the reference coordinate system is located in the center of a pre-defined first reference marker 24 and that the x-axis extends through this pre-defined first reference maker 24 and a pre-defined second reference marker 24.)

To facilitate a differentiation between object markers 14 and reference markers 24 in the images, they may have different optical characteristics (e.g., different forms). In other scenarios, the differentiation may be implicitly performed by the point cloud matching described above. In this manner, points associated with the object markers 14 will automatically be discriminated in the point cloud matching process described above. These discriminated "excess" points will then be attributed to object markers 14.

Once the position and orientation of the reference coordinate system 30 in the base coordinate system has been determined, the object marker positions are transformed from the base coordinate system to the reference coordinate system. This transformation process is illustrated in Fig. 3C for the individual object marker 14A, but will of course be repeated for each of the object markers 14 that are visible from the first viewing angle (i.e., the three object markers 14 exemplarily illustrated in the first image 26 of Fig. 3B).

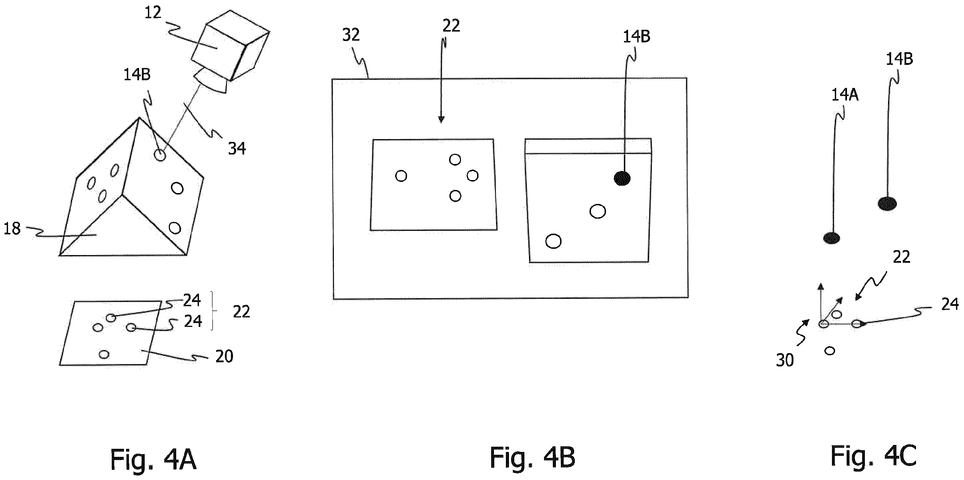

The positions of the remaining object markers 14 arranged on the second surface of the object 18 and, therefore, not visible from the first viewing angle cannot yet be reconstructed from the images captured from the first viewing angle. For this reason, the processing steps discussed above with reference to Figs. 3A to 3C will be repeated for (at least) a third image and a fourth image captured by the two camera modules, respectively, of the imaging device 12 from a second viewing angle. As illustrated in Fig. 4A, the second viewing angle is selected such that the second face of the object 14 is in the field of view of the imaging device 12, so that the images captured from the second viewing angle contain the remaining object markers 14 that could not yet be captured from the first viewing angle. Depending on the geometric properties of the object 18 and depending on where the object markers 14 have been placed on the object 18, there may arise the need to capture images from additional viewing angles so as to capture images that contain all image markers 14.

Fig. 4B exemplarily illustrates the content of the third image 32 captured by one of the camera modules of the imaging device 12 from the second viewing angle. The fourth image captured by the other camera module from the second viewing angle will be very similar to the third image 32, as explained above for the first image 26 and second image. It becomes apparent Form Fig. 4B that the third image 32 contains the remaining object markers 14 (such as object marker 14B) that were not in the field of view of the imaging device 12 when the first image 26 and the second image were captured from the first viewing angle (see Fig. 3B). Moreover, the third image 32 again contains the reference pattern 22 with all reference markers 24.

In a next step, the positions of the remaining object markers 14 and the reference markers 24 in the base coordinate system are determined, and then the positions of the remaining object markers 14 in the reference coordinate system 30 are reconstructed, as explained above. Finally, the object marker positions in the reference coordinate system 30 as determined from various viewing angles can be brought together, as shown in Fig. 4C, using the reference coordinate system 30 as an anchor. For bringing the object marker positions together, it is therefore required that the reference pattern 22 is contained in the images that are used for reconstructing the object marker positions.

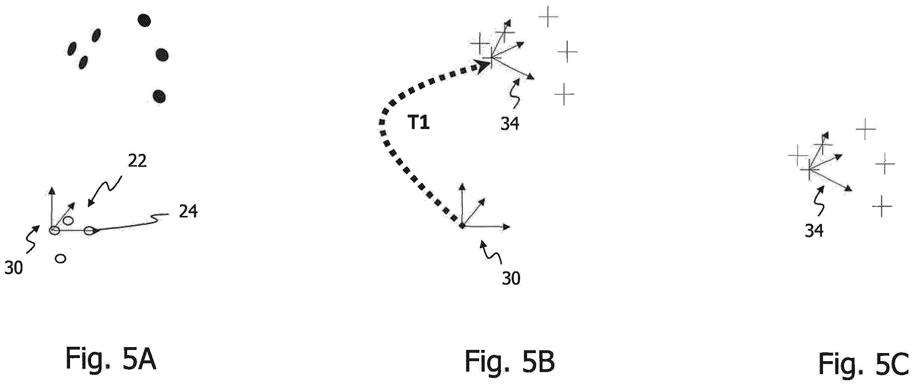

Fig. 4C exemplarily illustrates the position of the individual object marker 14A as determined from the first viewing angle, but also the position of the other object marker 14B as determined from the second viewing angle. In the end, the positions of all reference markers 14 in be reference coordinate system 30 will be known, as illustrated in Fig. 5A.

In a next step, the previously identified positions of the reference markers 24 are discarded (see Fig. 5B, in which the object marker positions are now denoted by crosses), and an object marker coordinate system 34 is defined. In the example illustrated in Figs. 5B and 5C, an origin of the object marker coordinate system 34 is chosen to coincide with the position of one of the object markers 14. An orientation of the object marker coordinate system 34 (e.g., of its x-axis) is defined by a line connecting the object marker position defining the origin with the position of a dedicated further object marker 14. As a result, a coordinate system transformation T1 between the reference coordinate system 30 and the object marker coordinate system 34 is obtained. This transformation T1 can be used during a tracking phase for registration purposes and may thus be stored locally by the processing device 10 or in a memory of the object 18. The object marker arrangement 16 (see Fig. 5C) can likewise be stored (and independently from the transformation T1 or the reference coordinate system 30), for example as a point cloud defined by the Euclidean distances between the object marker positions or as object marker coordinates in the object marker coordinate system 34.

In the example described above with reference to Figs. 3A to 5C, only four images (two images taken for each of two viewings angles) were used for determining the marker arrangement 16. In other examples, more than two images may be used, such as three, four, five, six or more images. Moreover, images may be taken from more than two viewing angles. Generally, a larger number of images and viewing angles increases the positional accuracies for the object markers 14, as statistical errors are reduced. However, not all images are required to contain all object markers 14, as explained above with reference to Figs. 3A to 4C. For example, the first image 26 and the second image contain the object marker 14A but not the object marker 14B. Similarly, the third image 32 and the fourth image contain the object marker 14B but not the object marker 14A.

Following the object marker arrangement determination (or "calibration") phase discussed above with reference to Figs. 2 to 5C, the object marker arrangement 16 defines a "rigid body" or "object tracker" that may be used for the purpose of tracking the object 18 in a later tracking phase.

The corresponding "object tracker" information may be stored in the processing device 10 or in the object 18 itself (e.g., in case the object 18 is a medical imaging device) for retrieval in the tracking phase. The tracking phase may take place days or weeks after the calibration phase. Moreover, while the calibration phase will only take place once (as long as the object markers 14 stay in place), repeated tracking phases may be executed making use of the "object tracker" information.

The reference device 20 was arranged in a fixed spatial relationship relative to the object markers 14 when the images were captured, in order to establish a common reference coordinate system 30 in space that can be used as anchor in combination with multiple different viewing angles. Once the images have been captured (and the positions of the object markers 14 have been determined), the reference device 20 may be removed from its spatial relationship with the object markers 14. Since the object marker coordinate system 34 and the object marker positions therein have been defined independently of the reference device 20, the object 18 can be tracked solely based on the object marker arrangement 16 after removal of the reference device 20. In other words, the reference device 20 is only needed in the calibration phase but not in the tracking phase. In some implementation, the reference device 20 may still be used in the tracking phase to track an object different from the object 18 carrying the marker arrangement 16. As such, a conventional (e.g., planar) tracking device may function as reference device 20.

In the tracking phase, the method illustrated in Fig. 2 may comprise a further step of receiving, by the processing device 10 or a dedicated tracking system, tracking image data representative of the object markers 14. The tracking image data may be captured by the same imaging device 12 (e.g., camera or camera modules) that captured the image data for the purposes of steps 102 and 104. Alternatively, the tracking image data may be captured using a different imaging device. When capturing the tracking image data, the imaging device 12 may remain stationary.

The method illustrated in Fig. 2 may further comprise tracking the object 18 based on the tracking image data and information about the object marker arrangement 16. The information about the object marker arrangement 16 (e.g., in terms of object marker positions in the object marker coordinate system 34) may be used to identify individual object markers 14 in the tracking image data and, thereafter, to determine a pose of the object marker coordinate system 34 (e.g., via point cloud matching, as generally described above). The pose of the object marker coordinate system 34 may be determined in a coordinate system of the imaging device 12. As the object 18 to be tracked moves in space, the pose of the object marker coordinate system 34 moves as well, and this pose movement can be tracked for visualization or navigation purposes (e.g., in a surgical navigation context).

For such visualization or navigation purposes, the method may further comprise registering a geometrical attribute of the object 18 with the object marker arrangement 16 or the object marker coordinate system 34. The geometrical attribute may comprise at least one of a rotation axis of the object 18, a position of an object tip (e.g., of a drill tip in case the object 18 is a surgical drill), at least a portion of a virtual model of the object 18 (e.g., of an icon visualizing a burr head in case the object 18 is a surgical burr), and a location of an imaging plane or imaging volume in case the object 18 is a medical imaging device (e.g., a CT scanner).

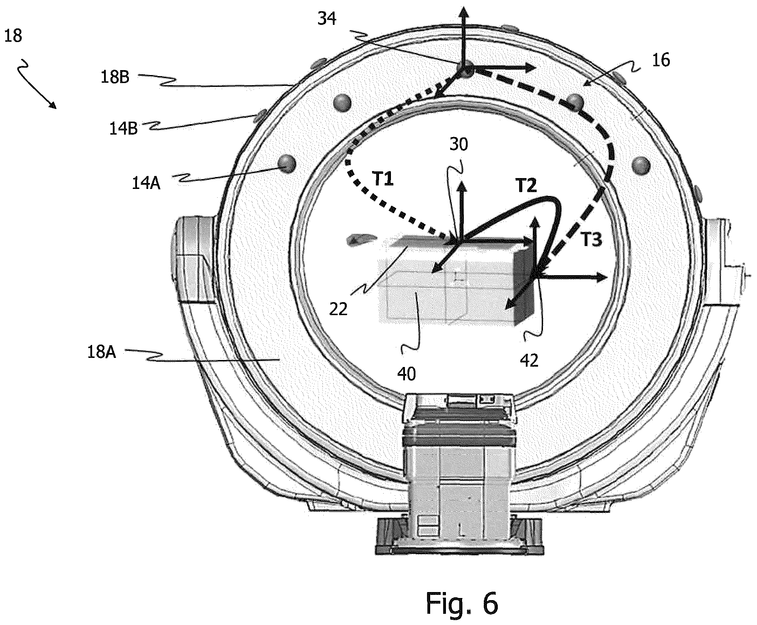

Fig. 6 illustrates an exemplary registration process for the Stryker Airo^{®} CT scanner that in one variant acts as the object 18 to be tracked. In such a tracking scenario, an imaging volume 40 of the CT scanner 18 having an associated imaging coordinate system 42 may be the geometrical attribute that needs to be tracked. As a prerequisite of the tracking procedure, the imaging coordinate system 42 has to be registered with the object marker coordinate system 34 in which the object marker arrangement 16 is defined.

In the non-limiting example of Fig. 6, a first set of object markers 14 including object marker 14A has been arranged on a planar front surface 18A of the CT scanner 18 and a second set of object markers 14 including object marker 14B has been arranged on a cylindrically curved side surface 18B of the CT scanner 18. The entirety of object markers 14 have been arranged by a user taking into account specific requirements and settings in the (e.g., operating) room in which the CT scanner 18 is placed. For example, these requirements and settings may define a preferred viewing angle of a tracking camera (not shown).

Prior to the registration process, the object marker arrangement 16 is determined (e.g., in terms of Euclidean distances between each pair of reference markers 14) as explained above with reference to Figs. 2 to 5C. In one variant, a first viewing angle (Figs. 3A to 3C) is associated with a front view onto the front surface 18A of the CT scanner 18 and a second viewing angle (Figs. 4A to 4C) is associated with a top view onto the upper side surface 18B of the CT scanner 18.

The registration of interest between the imaging coordinate system 42 and the object marker coordinate system 34 is defined by a coordinate system transformation denoted as T3 in Fig. 6. In the variant illustrated in Fig. 6, the transformation T3 is determined using the reference device 22 (that has previously been used to determine the object marker arrangement 16) as an anchor. In more detail, it is assumed that the reference device 22 was located in the imaging volume 30 when the object marker arrangement 16 has been determined. As part of the object marker arrangement determination process, the transformation T1 between the reference coordinate system 30 and the object marker coordinate system 34 has been determined (see Fig. 5B). Alternatively, the transformation T1 can be determined by the tracking camera based on images containing both the object marker arrangement 16 and the reference pattern 22, and based on geometrical information about the object marker arrangement 16 and the reference pattern 22 (e.g., the associated point clouds and information about the position and orientation of the respective coordinate system 34, 30).

The coordinate system transformation T3 is the product of the coordinate system transformation T1 and a coordinate system transformation T2 between the reference coordinate system 30 and the imaging coordinate system 42. This mathematical fact can be expressed as T3 = T1 x T2. Once the transformation T2 has been determined, the transformation T3 can be determined as well since the transformation T1 has already been determined earlier.

For determining the transformation T2 between the reference coordinate system 30 and the imaging coordinate system 42, the reference pattern 20 is configured such that each reference marker 24 is co-located with a reference fiducial that can be imaged by the CT scanner 18, or has a predetermined spatial relationship to such a reference fiducial. If, for example, the reference markers 24 are LEDs, the soldering points of the LEDs (that have a predetermined spatial relationship relative to an optical LED center) can act as reference fiducials since they will create artefacts in the CT images. If the reference markers 24 are reflective passive elements, a small lead ball may be located in the center of each reference marker 24 to create artefacts in the CT images. Therefore, the transformation T2 can be determined from such artefacts in the CT images and the predetermined relationship between the CT artefacts and the optical reference markers 24.

Once the transformation T2 has thus been determined, the transformation T3 can be calculated as T3 = T1 x T2. As soon as the transformation T3 is known, tracking of a movement of the object marker arrangement 16 (i.e., of the associated object marker coordinate system 34) in space will permit to determine the associated movement of the imaging volume (i.e., of the imaging coordinate system 42). During this tracking procedure, the reference device 22 is no longer needed and can be removed. In other variants, the reference device 22 may be realized by a patient tracker that is used for tracking patient movements relative to the imaging volume 40.

The technique described herein allows a user to build a customized object tracker based on a user-defined object marker arrangement 16 that initially is unkown to the processing device 10. For example, if a user wants to track a C-arm, the user can manually attach a plurality of object markers 14 to the C-arm at user-selected positions and then provide a nearby reference device 20. The user subsequently operates an imaging device 12 (such as a camera of a surgical navigation system or a webcam) to capture image data of the object markers 14 and the reference device 20, as explained above. Once a position of each object marker 14 has been determined based on the image data and based on geometrical information about the reference pattern 22 of the reference device 20, the customized object tracker can be built from the positional information thus obtained. The reference device 20 is no longer needed for tracking the object 18 and can be removed prior to the actual object tracking procedure.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A method for determining an object marker arrangement (16) comprising a plurality of object markers (14) arranged on at least two non-parallel surfaces or non-parallel surface portions of an object (18), wherein the object marker arrangement (16) is **characterized by** positions of the object markers (14), wherein a reference device (20) with a pre-determined reference pattern (22) is provided, the method comprising the following steps at least partially performed by a processing device (10):
receiving image data representative of a plurality of images that contain the reference pattern (22) and at least a subset of the object markers (14), wherein at least some of the images were captured by an imaging device (12) from different viewing angles, wherein the reference pattern (22) and the object markers (14) were arranged in a fixed spatial relationship relative to each other when the images were captured; and
determining the positions of the object markers (14) relative to the reference pattern (22), wherein the position of an individual one (14A) of the object markers (14) is determined based on at least two images that contain the individual object marker (14A) and based on geometrical information about the reference pattern (22).

2. The method according to claim 1, wherein
the different viewing angles comprise at least
- a first viewing angle in which the individual object marker (14A) is visible; and
- a second viewing angle in which the individual object marker (14A) is not visible but at least another one (14B) of the object markers (14) is visible.

3. The method according to any of the preceding claims, wherein
the image data have been taken by the imaging device (12) while the imaging device (12) was moving relative to the object marker arrangement (16).

4. The method according to any of the preceding claims, wherein
the imaging device (12) is a video camera capturing video data comprising the image data.

5. The method according to any of the preceding claims, wherein
determining the position of the individual object marker (14A) comprises
determining, based on at least a first image and a second image of the plurality of images that contain the reference pattern (22) and the individual object marker (14A), a position of the individual object marker (14A) relative to the reference pattern (22).

6. The method according to claim 5, wherein
another one (14B) of the individual object markers (14) is not contained in at least one of the first image and the second image, and further comprising determining, based on at least a third image and one of the first, the second and a fourth image of the plurality of images that contain the reference pattern (22) and the other one (14B) of the object markers (14), a position of the other one (14B) of the object markers (14A) relative to the reference pattern (22).

7. The method according to any of the preceding claims, wherein
at least one of the object markers (14) has a rotational-symmetric shape.

8. The method according to any of the preceding claims, wherein
the object marker arrangement (16) is determined in an object marker coordinate system (34), and wherein an origin of the object marker coordinate system has a predefined geometric relationship to one of the object markers (14).

9. The method according to any of the preceding claims, wherein
at least one of the object markers (14) comprises a reflective material configured to reflect light of at least one of the visible and infrared spectrum.

10. The method according to any of the preceding claims, further comprising
removing the reference device (20) from the fixed spatial relationship with the object markers (14); and
tracking the object (18) based on the object marker arrangement (16) after the reference device (20) has been removed.

11. The method according to any of the preceding claims, further comprising
receiving tracking image data representative of the object markers (14); and
tracking the object (18) based on the tracking image data and information about the object marker arrangement (16).

12. The method of any of the preceding claims, comprising
selecting the position of one of the object markers (14) as a reference position for an object marker coordinate system (34) and transforming the positions of the other object markers (14) in the object marker coordinate system (34).

13. The method of claim 12 in combination with at least one of claims 10 and 11, wherein
tracking the object (18) based on the object marker coordinate system (34).

14. The method according to any of the preceding claims, further comprising
registering a geometrical attribute of the object (18) with the object marker arrangement (16) or an object marker coordinate system (34).

15. The method according to any of the preceding claims, further comprising
manually arranging the object markers (14) on the object (18) at not-predefined positions before capturing the plurality of images.

16. The method according to claim 15, wherein
each object marker (14) is arranged via an adhesive or a magnetic force on the object (18).

17. A computer program product, comprising instructions that, when executed on at least one processor, cause the at least one processor to carry out any the methods of claims 1 to 16.

18. A processing device (10) for determining an object marker arrangement (16) comprising a plurality of object markers (14) arranged on at least two non-parallel surfaces or non-parallel surface portions of an object (18), wherein the object marker arrangement (16) is **characterized by** positions of the object markers (14), wherein a reference device (20) with a pre-determined reference pattern (22) is provided, wherein the processing device (10) is configured to
receive image data representative of a plurality of images that contain the reference pattern (22) and at least a subset of the object markers (14), wherein at least some of the images were captured by an imaging device (12) from different viewing angles, wherein the reference pattern (22) and the object markers (14) were arranged in a fixed spatial relationship relative to each other when the images were captured; and
determine the positions of the object markers (14) relative to the reference pattern (22), wherein a position of an individual one (14B) of the object markers (14) is determined based on at least two images that contain the individual object marker (14B) and based on geometrical information about the reference pattern (22).

19. The processing device (10) according to claim 18, further configured to perform the method according to any of the claims 2 to 16.

20. A system comprising the processing device of claim 18 or 19 and an imaging device (12) configured to capture the image data while moving relative to the object markers (14).

## Amended claims

### Amended claims in accordance with Rule 137(2) EPC.

1. A method for determining an object marker arrangement (16) comprising a plurality of object markers (14) arranged on at least two non-parallel surfaces or non-parallel surface portions of an object (18), wherein the object marker arrangement (16) is **characterized by** positions of the object markers (14), wherein a reference device (20) with a pre-determined reference pattern (22) is provided, the method comprising the following steps at least partially performed by a processing device (10):
receiving image data representative of a plurality of images that contain the reference pattern (22) and at least a subset of the object markers (14), wherein at least some of the images were captured by an imaging device (12) from different viewing angles, wherein the reference pattern (22) and the object markers (14) were arranged in a fixed spatial relationship relative to each other when the images were captured; and
determining the positions of the object markers (14) relative to the reference pattern (22), wherein the position of an individual one (14A) of the object markers (14) is determined based on at least two images that contain the individual object marker (14A) and based on geometrical information about the reference pattern (22);
determining, based on the determined positions of the object markers (14), the object-marker arrangement (16) on the at least two non-parallel surfaces or non-parallel surface portions of the object (18).

2. The method according to claim 1, wherein
the different viewing angles comprise at least
- a first viewing angle in which the individual object marker (14A) is visible; and
- a second viewing angle in which the individual object marker (14A) is not visible but at least another one (14B) of the object markers (14) is visible.

3. The method according to any of the preceding claims, wherein
the image data have been taken by the imaging device (12) while the imaging device (12) was moving relative to the object marker arrangement (16).

4. The method according to any of the preceding claims, wherein
the imaging device (12) is a video camera capturing video data comprising the image data.

5. The method according to any of the preceding claims, wherein
determining the position of the individual object marker (14A) comprises
determining, based on at least a first image and a second image of the plurality of images that contain the reference pattern (22) and the individual object marker (14A), a position of the individual object marker (14A) relative to the reference pattern (22).

6. The method according to claim 5, wherein
another one (14B) of the individual object markers (14) is not contained in at least one of the first image and the second image, and further comprising determining, based on at least a third image and one of the first, the second and a fourth image of the plurality of images that contain the reference pattern (22) and the other one (14B) of the object markers (14), a position of the other one (14B) of the object markers (14A) relative to the reference pattern (22).

7. The method according to any of the preceding claims, wherein
at least one of the object markers (14) has a rotational-symmetric shape.

8. The method according to any of the preceding claims, wherein
the object marker arrangement (16) is determined in an object marker coordinate system (34), and wherein an origin of the object marker coordinate system has a predefined geometric relationship to one of the object markers (14).

9. The method according to any of the preceding claims, wherein
at least one of the object markers (14) comprises a reflective material configured to reflect light of at least one of the visible and infrared spectrum.

10. The method according to any of the preceding claims, further comprising
removing the reference device (20) from the fixed spatial relationship with the object markers (14); and
tracking the object (18) based on the object marker arrangement (16) after the reference device (20) has been removed.

11. The method according to any of the preceding claims, further comprising receiving tracking image data representative of the object markers (14); and tracking the object (18) based on the tracking image data and information about the object marker arrangement (16).

12. The method of any of the preceding claims, comprising
selecting the position of one of the object markers (14) as a reference position for an object marker coordinate system (34) and transforming the positions of the other object markers (14) in the object marker coordinate system (34).

13. The method of claim 12 in combination with at least one of claims 10 and 11, further comprising:
tracking the object (18) based on the object marker coordinate system (34).

14. The method according to any of the preceding claims, further comprising
registering a geometrical attribute of the object (18) with the object marker arrangement (16) or an object marker coordinate system (34).

15. The method according to any of the preceding claims, further comprising
manually arranging the object markers (14) on the object (18) at not-predefined positions before capturing the plurality of images.

16. The method according to claim 15, wherein
each object marker (14) is arranged via an adhesive or a magnetic force on the object (18).

17. A computer program product, comprising instructions that, when executed on at least one processor, cause the at least one processor to carry out any the methods of claims 1 to 16.

18. A processing device (10) for determining an object marker arrangement (16) comprising a plurality of object markers (14) arranged on at least two non-parallel surfaces or non-parallel surface portions of an object (18), wherein the object marker arrangement (16) is **characterized by** positions of the object markers (14), wherein a reference device (20) with a pre-determined reference pattern (22) is provided, wherein the processing device (10) is configured to
receive image data representative of a plurality of images that contain the reference pattern (22) and at least a subset of the object markers (14), wherein at least some of the images were captured by an imaging device (12) from different viewing angles, wherein the reference pattern (22) and the object markers (14) were arranged in a fixed spatial relationship relative to each other when the images were captured; and
determine the positions of the object markers (14) relative to the reference pattern (22), wherein a position of an individual one (14B) of the object markers (14) is determined based on at least two images that contain the individual object marker (14B) and based on geometrical information about the reference pattern (22);
determine, based on the determined positions of the object markers (14), the object-marker arrangement (16) on the at least two non-parallel surfaces or non-parallel surface portions of the object (18).

19. The processing device (10) according to claim 18, further configured to perform the method according to any of the claims 2 to 16.

20. A system comprising the processing device of claim 18 or 19 and an imaging device (12) configured to capture the image data while moving relative to the object markers (14).
